# EUROPEAN PATENT SPECIFICATION

(11) **EP 0 403 779 B1**
(45) Date of publication and mention of the grant of the patent: **05.04.1995**
(21) Application number: 90108890.6
(22) Date of filing: 11.05.1990
(51) Int. Cl.: C08G 18/73, C08G 18/80, C09D 175/04, C09D 5/03

(54) **Curable powder coating compositions containing caprolactam-blocked isocyanates**
Härtbare Pulverbeschichtungszusammensetzungen, die caprolactamblockierte Isocyanate enthalten
Compositions de revêtement pulvérulentes durcissables contenant des isocyanates bloqués par caprolactame

(30) Priority: 19.06.1989 US 368472
(43) Date of publication of application: 27.12.1990
(73) Proprietor: CYTEC TECHNOLOGY CORP., Wilmington, Delaware 19801 (US)
(72) Inventor: Parekh, Girish Girdhar, Libertyville, Illinois 60048 (US)
(74) Representative: Diehl, Hermann O. Th., Dr.

(56) References cited:
- EP-A- 0 182 996
- EP-A- 0 218 040
- EP-A- 0 237 997
- EP-A- 0 263 983
- EP-A- 0 319 929
- WO-A-89/05320
- DE-A- 3 711 375
- US-A- 4 395 529

## Description

### BACKGROUND OF THE INVENTION

United States Patent No. 3,822,240 and WO-A1-89/05320 disclose the use of isophorone diisocyanate (IPDI), a commercially available diisocyanate in blocked form, as having utility for polyurethane powder coating processes. The caprolactam-blocked isophorone diisocyanate used in that invention is said to be a solid, melting at 53-55°C, and having a deblocking temperature of 175°C. The melting point of this material is too low for long-term stability and the deblocking temperature (or resistance to deblocking at a given temperature) is too high for rapid curing.

The lowest temperatures at which polyurethane powder coatings have been prepared in the prior art (US 3,822,240, for example) in a commercially useful manner has been 175°C. While this is a temperature that is suitable for some end users of powder coatings, it is wasteful of energy. Furthermore, exposure to unnecessarily high temperatures encourages irrepairable damage to the crosslinked films by introducing imperfections in the network and promoting excessive oxidation and yellowing. It is, therefore, highly desirable to carry out the curing process at as low a temperature as possible to avoid decomposition and other side reactions that have a negative impact on the film properties of powder coatings.

U.S. Patent No. 4,725,661 describes the preparation of blocked polyisocyanates. Among the listed possibilities of polyisocyanates is mentioned tetramethylxylylene diisocyanate (also named α,α,α',α'-tetramethyl xylylene diisocyanate, and hereinafter called TMXDI® aliphatic diisocyanate, product of American Cyanamid Company, Wayne, New Jersey). Among the possibilities for blocking agents is mentioned ε-caprolactam; however, the combination of the two is not exemplified.

While numerous uses of blocked m/p-TMXDI have been disclosed in the patent literature, none has dealt with the uses of blocked m/p-TMXDI in powder coatings. Thus, U.S. Patent No. 4,725,661 discloses heat-curing, one pack resin compositions comprising blocked TMXDI or TMXDI-based prepolymers, without mention of uses in powder coatings. Similarly, US 4,748,200 discloses blocked TMXDI applications in electrodeposition compositions.

British patent 2,205,322 describes the use of blocked TMXDI and prepolymers in coating metals, but no mention is made of powder coating applications.

German patent DE 3,711,375 describes half-blocked TMXDI and derivatives, without any mention of the potential uses in powder coatings.

The above disclosures of various applications of blocked TMXDI and related prepolymers involve blocked systems that are not crystalline powders, and therefore are unsuitable for powder coatings applications. This is the case because powder technology requires the curable compositions used in powder coating to be in the solid state and to remain in the solid state at temperatures up to about 50°C., but become fluid at about 150°C, forming low viscosity melts.

It is desirable, therefore, to prepare a blocked isocyanate powder coating composition with a higher melting point and a faster deblocking rate to have bettor performing compositions for powder coating applications.

### FIELD OF THE INVENTION

This invention relates to curable compositions useful for powder coating systems.

### SUMMARY OF THE INVENTION

This invention is a curable powder coating composition comprising:
(i) an adduct of meta- and/or para-tetramethylxylylene diisocyanate with two equivalents of ε-caprolactam and
(ii) a solid powdered isocyanate-reactive polyfunctional material which is solid at ambient temperature, and
wherein the ratio of ingredients (i)/(ii) is from 0.5 to 2, and the sum of ingredients (i) and (ii) constitutes at least 30 weight percent of the total weight of the composition.

This invention is also an improved method of coating substrates by applying to substrates the coating composition of the invention.

This invention is also a crosslinked article or coating formed from heat-activating and curing the urethane forming powder coating compositions of this invention.

### THE DRAWINGS

Figure 1 shows two plots of viscosity vs. temperature for a polyester resin formulation containing the crosslinker adduct of the invention and the same polyester resin formulation containing a commercially available control crosslinker.

Figure 2 shows two plots of the viscosity vs. temperature for an acrylic resin formulation containing the crosslinker adduct of the invention and the same acrylic resin formulation containing a commercially available control crosslinker.

### DETAILED DESCRIPTION OF THE INVENTION

### THE CURABLE POWDER COATING COMPOSITIONS OF THE INVENTION:

The novel low temperature curable powder coating composition of the invention comprises as essential ingredients; (i) TMXDI® diisocyanate blocked with e-caprolactam (hereinafter TMXDI/e-caprolactam), and (ii) a solid powdered isocyanate-reactive polyfunctional material.

### A. Nature and Preparation of the TMXDI/e-caprolactam Ingredient:

The TMXDI/e-caprolactam blocked isocyanate ingredient is easily prepared by combining TMXDI diisocyanate with approximately two equivalents of e-caprolactam under conditions which permit reaction. Half-blocked polyisocyanates do not permit the preparation of stable powder coating compositions and are not within the scope of this invention.

The TMXDI® diisocyanate used in the preparation of the adduct ingredients for the compositions of this invention may be any one of the isomers of meta- or para-TMXDI or mixtures thereof. The meta-adduct of TMXDI is generally preferred because of commercial availability. The preparation of TMXDI itself is described in U.S. Patent Nos. 3,290,350, 4,130,577, and 4,439,616.

Typically, the TMXDI/e-caprolactam adduct formation is effected in a non-isocyanate reactive solvent such as toluene, benzene, xylene, decane and the like or mixtures thereof. The blocking reaction is generally performed at above ambient tempratures, and preferably at from about 50°C. to about 110°C. Adduct formation may be enhanced by the presence of small amounts of a organotin-based catalyst such as tetrabutyl diacetoxystannoxane (TBDAS) at about 0.1% loading relative to the weight of TMXDI. The time for adduct formation is not critical and is usually less than about 24 hours. 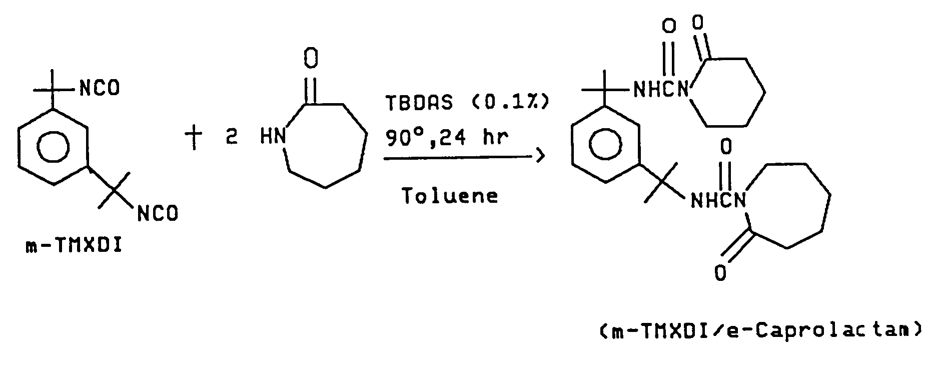
The product TMXDI/e-caprolactam adduct separates on cooling with no requirement for further purification. The blocking reaction of TMXDI with e-caprolactam and the properties of the TMXDI/e-caprolactam adduct are given below.

### Properties of the TMXDI/e-caprolactam adduct:

| | |
|---|---|
| Appearance: | White, crystalline solid |
| melting point | 115-126°C |
| molecular weight | 470 |
| equivalent weight (-NCO): | 235 |
| Compatible with : | Acrylic resins |
| | Polyester resins |
| Solvents: | butyl acetate |
| | methyl ethyl ketone |
| | methoxypropyl acetate |
| | methyl isobutyl ketone |
| Cure Temperature: | 150-190°C/20 min |
| Catalyst: | TBDAS |

### B. The Solid Powdered Isocyanate-Reactive Material:

The TMXDI/e-caprolactam adduct described in the preceding section constitutes one of the two necessary components required for the curable compositions of this invention. The other necessary component is a solid powdered isocyanate-reactive material such as a polyfunctional reactant containing functional groups that are capable of reacting with the isocyanate functions generated by the process of thermal deblocking the TMXDI/e-caprolactam adduct. The isocyanate reactive functional groups are selected from the set comprising; amino groups, carboxyl groups, mercapto groups, hydroxy groups, or combinations of any of the aforementioned groups. Isocyanate reactive materials having a plurality of hydroxy groups are particularly preferred in the practice of the invention.

Suitable polyfunctional hydroxy reactant materials may be acrylic resins containing pendant hydroxy functionalities, or polyester resins with terminal hydroxy groups, which are described in more detail below.

Resins used in the formulation of this invention are intended for powder coating applications and must be solid at ambient temperatures and should remain solids up to about 50°C to preserve the powdery state of the curable composition and prevent lumping. Upon heating, however, these resins should become fluid to permit formation of low viscosity melts which allow the components of the curable composition to flow evenly over the substrate prior to the deblocking and crosslinking process. It is desirable that the isocyanate reactive resins become fluid in a temperature range from about 100°C to about 150°C, and most preferably in the range from 120°C.to 140°C. The isocyanate reactive materials used in the composition of the invention preferably have an average molecular weight of from about 100 about 50,000.

### 1. ACRYLIC RESIN ISOCYANATE-REACTIVE MATERIAL

The hydroxyfunctional acrylic resins used in formulating the curable compositions of this invention preferably have molecular weights in the range of from about 1,000 to 50,000 and hydroxyl group equivalent weights of from about 200 to 5,000. An example of a a suitable acrylic resin is SCX-800 A{tm} acrylic resin, a product of S.C. Johnson and Sons, Inc. having the following physical and chemical properties:

| | |
|---|---|
| Hydroxyl value: | 43 |
| Equivalent Weight: | 1300 |
| Glass Transition Temperature (Tg): | 43°C |
| Softening Point: | 100°C |
| Acid Value: | 15 |

Commercially available acrylic resins are generally applicable for use in preparing the compositions of the invention. Selection of the optimum acrylic resin will depend of the users desire to impart to the final cured product particular physical properties such as hardness, softness, flexure, chemical/solvent resistance, and etc.

### 2. POLYESTER RESINS

The hydroxy functional polyester resins used in formulating the curable compositions of this invention preferably have molecular weights in the range of from about 1,000 to 50,000 and hydroxyl group equivalent weights of from about 200 to 10,000. An example of a suitable polyester resin is Cargill 3000 polyester, a product of Cargill Corporation, having the following physical and chemical properties:

| | |
|---|---|
| OH Number: | 50 |
| Equivalent Weight: | 1120 |
| Glass Transition Temperature (Tg): | 56°C |
| Acid Value: | <15 |

Upon cure, polyester resins give polyurethane films of superior properties, as described in detail in Example 2. A wide variety of commercial polyester resins may be used for the isocyanate reactive ingredient of the invention, provided such resins have the melting range and physical properties described above.

### 3. OTHER ISOCYANATE - REACTIVE SYSTEMS

Other polyhydroxy functional compounds solid at about 50°C or higher which are useful in the practice of the Invention are triols, tetraols, and higher polyhydroxy alcohols. Illustrative polyhydric alcohols are pentaerythritol (m.p. 262°C), dipentaerythritol, tripentaerythritol, trimethylolethane, trimethylolpropane, annhydroenneahepititol (RN 4744-47-2), 1,2,4-betanetriol, and 1,2,6-hexanetriol. These polyols can also be used to totally or partially replace the acrylic resin or polyester resin isocyanate reactive ingredient of the composition of the invention. However, the film properties resulting from addition of such non-resinous ingredients sometimes are inferior and lack flexibility in the cured film final product. Preferably, relatively low molecular weight polyhydroxy functional compounds may be used advantageously in combination with hydroxy functional acrylic or polyester resins.

Polyfunctional amines are also suitable as isocyanate-reactive components, provided they are solids up to 50-60°C to allow storage stable powders containing the block isocyanates of this invention. The crosslinked films and articles resulting from amine-containing curable compositions, however, do not give polyurethanes. Instead amines form polyureas having physical properties somewhat different than polyurethanes but still useful in the same applications as polyurethanes.

Polyfunctional mercaptans produce polymeric thiocarbamates which also have different physical properties than polyurethanes and can be used in applications where a lower refractive index product is desirable.

### C. THE RATIO AND PROPORTION OF ESSENTIAL INGREDIENTS IN THE CURABLE COMPOSITION:

The TMXDI/e-caprolactam ingredient is combined with the isocyanate reactive ingredient in proportions ranging between 0.5 to 2.0 equivalents of the blocked isocyanate group per each equivalent of isocyanate reactive functionality on the isocyanate reactive material.

The TMXDI/e-caprolactam ingredient and the solid powdered isocyanate-reactive material together should constitute at least about 30 weight percent of the total weight of the composition.

Other curable powder coating composition ingredients may include small amounts of benzoin (viz., about 0.5 weight percent) as an additive. The composition may also include as a flow control agent ingredients such as Modaflow® Powder III Resin Modifier, a product of Monsanto Company. The curable composition may also include an organotin catalyst such as tetrabutyldiacetoxystannoxane for the purpose of accelerating cure rate. Moreover, fillers and pigments such as titanium dioxide based pigments may be conventionally added to improve the appearance and properties of the coatings and articles formed from the composition.

The curable compositions of this invention are believed to cure within about 20 minutes, a rate comparable to compositions disclosed in US 3,822,240. However, they do so at cure temperatures that are consistently 20-25°C lower than those disclosed in prior art giving film properties that are as good or better than those disclosed previously.

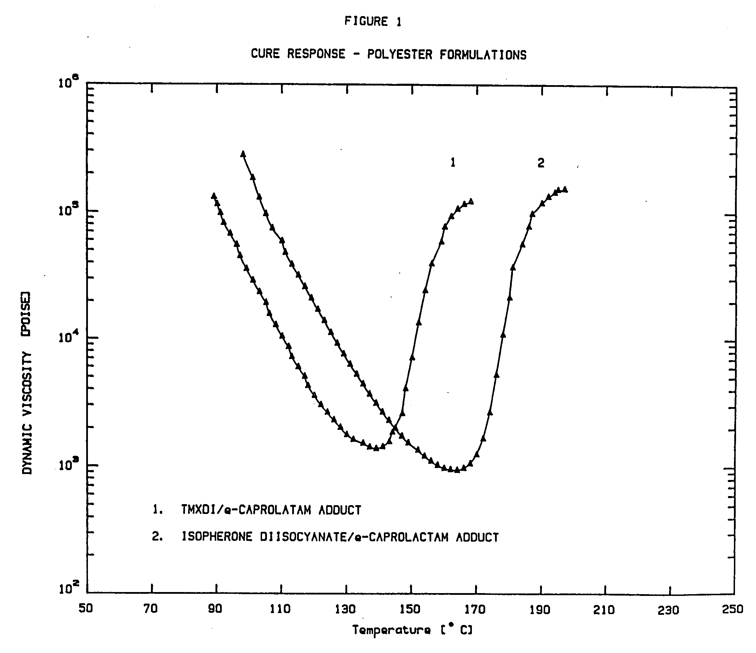

For example, monitoring the viscosity of a typical curable composition of this invention with that of an example of prior art showed clearly that the composition containing TMXDI/e-caprolactam adduct and hydroxy terminated polyester resin had an onset temperature for crosslinking at about 140°C, about 20°C lower than that of the prior art consisting of isophorone diisocyanate blocked with e-caprolactam and hydroxy terminated polyester resin combination (see Figure 1).

### D. THE IMPROVED METHOD OF COATING BY THIS INVENTION:

This invention is also an improved method of coating substrates by applying to substrates the coating composition of the invention.

"Powder coating" is an art recognized process and is defined herein as a method of electrostatic powder spraying wherein a finely divided solid coating material is electrostatically attracted to a surface of an article. The article covered with the powder is heated to at least the fusion temperature of the coating composition forcing it to flow out and form a coating which is cured by further application of heat.

The prior art powder coating process can be considerably improved by using as the coating material a low temperature heat-curable urethane coating composition comprising as essential ingredients; (i) m/p-TMXDI blocked with e-caprolactam, and (ii) a solid powdered isocyanate-reactive polymeric material. The unexpectedly superior properties of the powder coating compositions of the invention are their very low deblocking and crosslinking temperatures which offer a savings of energy and reduced thermal degradation of the final cured polymer product.

A typical procedure for using the compositions of the invention is as follows: The substrates are coated with the composition of the invention and subjected to a temperature of from about 120°C. to about 250°C to melt and spread the composition uniformly onto the substrate surface. Thereafter, the substrate is held at a temperature of from about 150°C to about 250°C to crosslink the composition and form the permanently cured coating.

### E. CROSSLINKED ARTICLES AND COATINGS FORMED BY THE COMPOSITIONS OF THIS INVENTION:

After thermal deblocking the reaction product of the unblocked isocyanate and hydroxy groups is a urethane group and because of the polyfunctional nature of the reactants, the resulting product in this case is a crosslinked polyurethane taking the form of a film such as in coatings or the form of an article such as in molded products.

Conventional crosslinkers for resins have a functionality greater than two. It is an unexpected finding of this invention that difunctional crosslinkers possess utility and advantageous properties.

The heat-cured compositions of this invention may be employed as coatings for wire, appliances, automotive parts, furniture, pipes, machinery, and the like. Suitable surfaces include metals such as steel aluminum, plastic, or plastic coated with a conductive primer.

The heat-cured compositions may also be used to form solid articles such as cases, enclosures, and structural members.

The following Examples illustrate various embodiments of the invention.

### EXAMPLE 1

This Example illustrates the preparation of m-TMXDI blocked e-caprolactam used as one of the essential ingredients in the powder coating composition of the invention:
m-TMXDI (195.4 g, 0.800 mole) was added to a solution of e-caprolactam (199.0 g, 1.76 mole) and tetrabutyl diacetoxystannoxane (0.200 g) in toluene (1000 ml) and the resulting mixture was heated to 90° for 24 hours. After cooling the mixture in an ice water bath, the first crop of m-TMXDI blocked e-caprolactam separated as colorless crystals (232.4 g, m.p. 125°C). Further cooling gave two additional crops (98.0 g, m.p. 118°C) bringing the total yield to 88%. The infrared and nuclear magnetic resonance spectra confirmed that the product was m-TMXDI blocked e-caprolactam 1:2 adduct.

Some of the physical properties of the m-TMXDI blocked e-caprolactam are summarized below:

| | |
|---|---|
| Appearance: | white crystalline solid |
| Melting Point | 115-126°C |
| Molecular Weight | 470 |
| Equivalent Weight (-NCO): | 235 |
| Compatible with: | acrylics |
| | polyesters,. |
| Cure temperature (°C) | 150-190°C/20 min |
| Catalyst: | TBDAS |

### EXAMPLE 2

### Preparation of Powder Coating Formulation and its Cure using a hydroxyl-bearing Polyester Resin

### Ingredients:

| Ingredient | Parts by weight |
|---|---|
| Cargill 3000 Polyester Resin* | 114.5 |
| m-TMXDI blocked e-caprolactam (prepared according to Example 1) | 27.5 |
| Benzoin (additive) | 1.2 |
| Modaflow 2 (flow control agent)** | 1.0 |
| TBDAS (organo-tin catalyst) | 1.4 |
| (Ti02 Pigment) | 57.0 |

| | |
|---|---|
| * = Carginn, Inc., Chemical Products Div., Cottage Avene, Carpentersville, IL 60110 | |
| ** = Modaflow®, Powder III Resin Modifier, Monsanto Company, 800 N. Lindbergh Bldv., St. Louis, MO 63167 | |

### Preparation and Curing of the Powder Coating Composition:

All components were dry blended in a Waring Blender for small runs. For larger scale runs, a Welex Mixer was used. The dry blended powder was then melt-mixed at 90°C. to 125°C for 1-5 minutes in a two roll mill to allow homogenous mixing without allowing significant crosslinking to occur. The melt-mixed material was then chopped into smaller pieces in a roll mill or a pelletizer, and fed into a mill classifier where the chips were ground to a fine powder with particle size less than 75 »m to allow preparing films with thickness less then 30 »m. The finely ground powder, charged with up to 80 kV power, was then sprayed through an electro-static spray gun on grounded metal substrate in a spray booth. The metal substrate was then baked in an oven at 150-200°C to allow the powder to fuse, flow out and crosslink. These processes were analyzed by means of a viscometer and a rheometer.

### Physical Properties of the Powder Coating Composition of this Example:

| | |
|---|---|
| Resin/Crosslinker Weight Ratio | 81/19 |
| Pigment/Binder Resin Weight Ratio | 0.4 |
| Gel Time at 200°C (sec.) | 50 |
| Vertical Plate Flow at 190°C. (cm) | 7.8 |

Film Properties of the cured Powder Coating Composition of this Example:

| | | Run 1 | Run 2 |
|---|---|---|---|
| Bake Temperature (°C) | | 175 | 190 |
| MEK Rubs | | 200+ | 200+ |
| Film thickness | (mm) | 0.051 | 0.051 |
| | (mils) | 2.0 | 2.0 |
| KHN (Knoop Hardness) | | 11.6 | 12.6 |
| Pencil Hardness | | 5H | 5H |
| Impact F/R* | (N) | 177.5-200.1/22.2-44.5 | 110.8-133.4/22.2-44.5 |
| | (lbs.) | 40-45/5-10 | 25-30/5-10 |
| Color** | | -0.04 | -0.04 |
| Gloss, 60°C*** | | 91 | 93 |
| Gloss, 20°C | | 65 | 67 |
| Salt Spray (500 hrs)**** | | No Change | No Change |
| Humidity (60°C, 21 Days) | | No Change | No Change |

| | | | |
|---|---|---|---|
| * = Front/Reverse | | | |
| ** = Tristimylus method | | | |
| *** = "Gloss" as measured by Glossmeter instrument manufactured by Hunter Labs, Reston, Virginia | | | |
| **** = using ASTM Test Method B-117 | | | |

This Example describes the preparation of a powder coating composition using the techniques of dry blending, melt mixing, pelletizing, mill classification. Thereafter the composition was applied by electrostatic powder spray techniques and subsequently cured at 150-250°C temperature range for about 20 minutes to form a cured composition within the scope of this invention.

### EXAMPLE 3

### Preparation of Powder Coating Formulation and its Cure using a hydroxyl-functional Acrylic Resins

### Ingredients:

| Ingredient | Parts by weight |
|---|---|
| SCX-800A Acrylic Resin* | 256 |
| m-TMXDI blocked e-caprolactam (prepared from Example 1) | 64 |
| Benzoin (additive) | 2 |
| Modaflow 2 (flow control agent) | 4 |
| TBDAS organo-tin catalyst) | 4 OR 650 |
| (Ti02 Pigment) | 80 |

| | |
|---|---|
| * = a hydroxy functional acrylic resin having (i) hydroxyl value of 43 ± 4; (ii) equivalent weight of 1300 | |

### B. Preparation and Curing of the Powder Formulation with TMXDI/e-caprolactam adduct/SCX-800A

The powder formulation was prepared and cured according to the procedure described in Example 2.

### C. Physical Properties of the Powder Formulation with TMXDI/e-caprolactam adduct/SCX-800A

| | |
|---|---|
| Resin/Crosslinker Ratio: | 80/20 |
| Pigment/Binder Ratio: | 0.25 |
| Gel Time at 200°C (sec.) | 33 |
| Vertical Plate Flow at 190°C (cm.) | 10.4 |

### D. Film Properties of TMXDI/e-caprolactam adduct/SCX-800A in Powder Coatings

| | | | |
|---|---|---|---|
| Bake Temperature (°C) | | 175 | 190 |
| MEK Rubs | | 10/200+ | 10/200+ |
| Film thickness | (mm) | 0.051 | 0.051 |
| | (mils) | 2.0 | 2.0 |
| KHN (Knoop Hardness) | | 15.4 | 16.2 |
| Pencil Hardness | | 4H | 4H |
| Impact F/R | (N) | 22.2-44.5/0-8.9 | 22.2-44.5/0-8.9 |
| | (lbs.) | 5-10/0-2 | 5-10/0-2 |
| Color (Tristimulus) | | -0.05 | -0.05 |
| Gloss, | 60°C | 68 | 72 |
| | 20°C | 35.5 | 31 |
| Salt Spray (240 hrs) | | No change | No change |
| Humidity (60°C, 30 Days) | | Microblisters | Microblisters |

### EXAMPLE 4

### Comparison of TMXDI/e-caprolactam adduct with Isopherone Diisocyanate blocked with e-caprolactam

A commercial blocked isocyanate from Cargill, CR2400 (Isopherone Diisocyanate blocked with e-caprolactam) , was used in the formulation of Example 2, Part A, to prepare a powder coated film. This film was compared with a powder coated film prepared according to the procedure of Example 2, part B using TMXDI/e-caprolactam adduct. Cargill 3000 hydroxy terminated polyester was used in both cases so that the observed differences in the film properties reflect the performance of TMXDI/e-caprolactam adduct and CR2400 blocked isocyanates only. The curing conditions and film properties of the two blocked isocyanates, TMXDI/e-caprolactam adduct and CR2400, are listed below:

### Film Properties of Powder Coatings from CR2400 and BI-10

| | | | |
|---|---|---|---|
| Blocked Isocyanate | | CR2400 | BI-10 |
| Structure | | IPDI-e-Caprolactam | m-TMXDI-e-Caprolactam |
| Resin | | Cargill 3000 Polyester | Cargill 3000 Polyester |
| Substrate | | Bonderite 100 | Bonderite 100 |
| Film Thickness | (mm) | 0.051/0.061 | 0.043/0.056 |
| | (mils) | 2.0/2.4 | 1.7/2.3 |
| Bake Cycle | | 190°C/20 min. | 190°C/20 min. |
| Film Appearance | | Orange Peel, Slight | Orange Peel |
| MEK Rubs | | 100+ | 100+ |
| KHN (Knoop Hardness) | | 14.6 | 12.6 |
| Pencil Hardness | | 2H+ | 2H+ |
| Impact F/R* | (N) | 200.1-222.7/44.5-88.9 | 110.8-133.4/22.2-44.5 |
| | (lbs.) | 45-50/10-20 | 25-30/5-10 |
| Color (Tristimulus) | | Not available | -0.04 |
| Gloss, | 60o | 93 | 93 |
| | 20o | 87 | 67 |
| Salt Spray | | 8(500 hrs) | 10(1000 hrs) |
| Humidity (60°C, 21 Days) | | No change | No change |

| | | | |
|---|---|---|---|
| *F/R = Front/Reverse | | | |

### EXAMPLE 5

The Isopherone Diisocyanate blocked with e-caprolactam and TMXDI/e-caprolactam adduct formulations of Example 4 were heated in a rheometer and the viscosity of the curing composition versus the temperature of the system was recorded. The results are depicted in Figure 1.

It is evident from the plot in Figure 1 that formulation 1 containing TMXDI/e-caprolactam adduct and Cargill 3000 Polyester Resin has an onset temperature of crosslinking at -140°C. In contrast, formulation 2 containing CR2400 has its onset temperature of crosslinking at -160°C, about 20°C higher than formulation 1 which contains TMXDI/e-caprolactam adduct.

It is concluded from these results, that TMXDI/e-caprolactam adduct deblocks advantageously at a lower temperature than CR2400, the leading commercial blocked isocyanate system.

### EXAMPLE 6

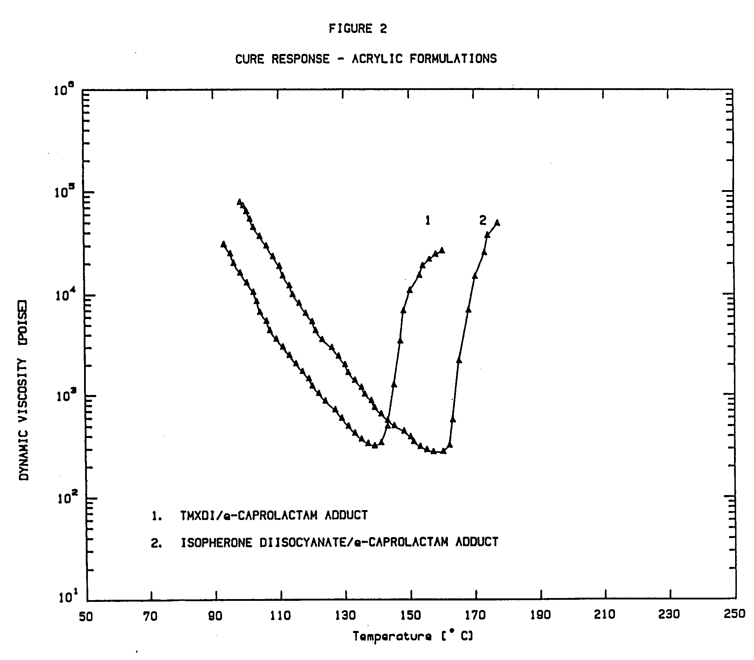

This Example was conducted using the procedure of Example 5 but using SCX-800A acrylic resin instead of the hydroxy terminated polyester resin. The results are depicted in Figure 2.

To demonstrate that the lowering of the onset temperature for crosslinking was not caused by the nature of the isocyanate reactive component, a similar comparison was made using a different isocyanate reactive material, SCX-800A Acrylic Resin instead of the Cargill 3000 polyester of Example 5. The results obtained were identical with those of Example 5, namely that TMXDI/e-caprolactam adduct/SCX-800A had an onset temperature for crosslinking at about 20°C. lower than that of CR2400/SCX-800A composition.

Again, it is concluded in this case as well, that formulations containing TMXDI/e-caprolactam adduct have lower onset temperatures for crosslinking then CR2400 in similar powder coating formulations.

### EXAMPLE 7

### Comparison of Rucote 107* Polyester Powder Coating Resin Polyester Cured with TMXDI/e-caprolactam Adduct in Powder Coatings Formulations

A powder coating formulation with TMXDI/e-caprolactam adduct was prepared according to the procedure described in Example 2, Part A and cured according to the procedure described in Example 2, Part B. A second formulation was also prepared by the procedure described in Example 2, Part A, except for replacement of Cargill 3000 resin with Rucote 107. The resulting composition was cured according to the procedure described in Example 2, Part B, as before. The physical properties and film properties are summarized below to allow direct comparison of the two resins, Rucote 107 and Cargill 3000, in TMXDI/e-caprolactam adduct cured powder coatings.
* A product of RUCO Polymer Corporation, New South Rd. Hicksville, Ny 11802 having the following properties: (i) OH Number: 47; (ii) Acid Number: 13; (iii) Glass Transition Temperature: 58°C; (iv) Equivalent Weight: 1190.

### Comparison of Properties Resulting from Different Resins in TMXDI/e-caprolactam adduct Cured Powder Coatings

| | | | | | |
|---|---|---|---|---|---|
| Blocked Isocyanate Structure | | m-TMXDI/e-Caprolactam | | n-TMXDI/e-Caprolactam | |
| Resin | | Rucote 107 | | Cargill 3000 | |
| Substrate | | Bonderite 100 | | Bonderite 100 | |
| R/X: | | 81/19 | | 81/19 | |
| P/B: | | 0.4 | | 0.4 | |
| Gel Time at 200°C (sec.) | | 76 | | 50 | |
| Vertical Plate Flow at 190°C (cm.) | | 7.7 | | 7.2 | |
| Bake Cycle | | 175°C | 190°C | 175°C | 190°C |
| MEK Rubs | | 200+ | 200+ | 200+ | 200+ |
| KHN (Knoop Hardness) | | 13.1 | 13.2 | 14.5 | 15.0 |
| Film Thickness | (mm) | 0.051 | 0.051 | 0.055 | 0.053 |
| | (mils) | 2.0 | 2.0 | 2.2 | 2.1 |
| Impact F/R* (lbs.) | | 120-130 | 120-125 | 80-85 | 80-85 |
| | | 5-10 | 15-20 | 5-10 | 10-20 |
| Gloss, | 60o | 93 | 93 | 92 | 92 |
| | 20o | 72 | 77 | 60 | 58 |

| | | | | | |
|---|---|---|---|---|---|
| * F/R = Front/Reverse | | | | | |

This Example illustrates that a variety of hydroxy functional isocyanate reactive materials have utility in the composition of the invention.

## Claims

1. A curable powder coating composition comprising:
(i) an adduct of meta- and/or para-tetramethylxylylene diisocyanate with two equivalents of ε-caprolactam and
(ii) a solid powdered isocyanate-reactive polyfunctional material which is solid at ambient temperature, and
wherein the ratio of ingredients (i)/(ii) is from 0.5 to 2 equivalents of the blocked isocyanate group per each equivalent of isocyanate reactive functionality on the isocyanate reactive material, and the sum of ingredients (i) and (ii) constitutes at least 30 weight percent of the total weight of the composition.

2. The composition of claim 1, wherein there are also contained normal additives selected from the group consisting of fillers, pigments, benzoin, flow control agents, and organotin catalysts.

3. The composition of claim 1 or 2, wherein the isocyanate reactive material contains reactive groups selected from hydroxy, amino and mercapto groups, and mixtures thereof.

4. The composition of claim 3, wherein the isocyanate reactive material contains a plurality of hydroxy groups.

5. The composition of claim 4, wherein the isocyanate reactive material is selected from a hydroxy functional acrylic resin and a hydroxy terminated polyester resin.

6. The composition of any one of claims 1 to 5 wherein the isocyanate reactive material has a molecular weight of from 100 to 50000.

7. The composition of any one of claims 1 to 6, wherein the solid powdered isocyanate-reactive polyfunctional material is solid up to 50°C and becomes fluid in a temperature range of from 100°C to 150°C.

8. A method of coating a substrate by contacting the substrate with a powder coating composition and thereafter heat curing said composition, characterized in that the powder coating composition is a powder coating composition according to any one of claims 1 to 7.

9. An adduct of meta- and/or para-tetramethylxylylene diisocyanate with two equivalents of ε-caprolactam.

## Patentansprüche

1. Härtbare Pulverbeschichtungszusammensetzung, umfassend:
(i) ein Addukt von Meta- und/oder Para-Tetramethylxylylendiisocyanat mit zwei Äquivalenten ε-Caprolactam und
(ii) ein festes, pulverförmiges, isocyanatreaktives, polyfunktionales Material, das bei Umgebungstemperatur fest ist, und
wobei das Verhältnis der Bestandteile (i)/(ii) 0,5 bis 2 Äquivalente der blockierten Isocyanatgruppe pro Äquivalent isocyanatreaktiver Funktionalität auf dem isocyanatreaktiven Material beträgt, und die Summe der Bestandteile (i) und (ii) zumindest 30 Gewichtsprozent des Gesamtgewichts der Zusammensetzung ausmacht.

2. Zusammensetzung nach Anspruch 1, wobei auch normale Additive enthalten sind, ausgewählt aus der Gruppe bestehend aus Füllstoffen, Pigmenten, Benzoin, Verlaufmitteln und Organozinnkatalysatoren.

3. Zusammensetzung nach Anspruch 1 oder 2, wobei das isocyanatreaktive Material reaktive Gruppen enthält, ausgewählt aus Hydroxy-, Amino- und Merkaptogruppen sowie Mischungen derselben.

4. Zusammensetzung nach Anspruch 3, wobei das isocyanatreaktive Material mehrere Hydroxygruppen enthält.

5. Zusammensetzung nach Anspruch 4, wobei das isocyanatreaktive Material aus einem hydroxyfunktionalen Acrylharz und einem hydroxyterminierten Polyesterharz ausgewählt ist.

6. Zusammensetzung nach einem der Ansprüche 1 bis 5, wobei das isocyanatreaktive Material ein Molekulargewicht von 100 bis 50000 aufweist.

7. Zusammensetzung nach einem der Ansprüche 1 bis 6, wobei das feste, pulverförmige, isocyanatreaktive, polyfunktionale Material bei Temperaturen bis zu 50°C fest ist und in einem Temperaturbereich von 100°C bis 150°C fluid wird.

8. Verfahren zur Beschichtung eines Substrats durch Kontaktierung des Substrats mit einer Pulverbeschichtungszusammensetzung und nachfolgender thermischer Härtung der Zusammensetzung, dadurch gekennzeichnet, daß es sich bei der Pulverbeschichtungszusammensetzung um eine Pulverbeschichtungszusammensetzung nach einem der Ansprüche 1 bis 7 handelt.

9. Addukt von Meta- und/oder Para-Tetramethylxylylendiisocyanat mit zwei Äquivalenten ε-Caprolactam.

## Revendications

1. Composition de revêtement en poudre durcissable, comprenant:
(i) un composé d'addition de méta- et/ou de para-tétraméthylxylylène diisocyanate avec deux équivalents d'ε-caprolactame, et
(ii) une substance polyfonctionnelle solide en poudre, réactive avec un isocyanate, qui est solide à la température ambiante, et
dans laquelle le rapport des ingrédients (i)/(ii) est de 0,5 à 2 équivalents du groupe isocyanate bloqué par équivalent de fonctionnalité réactive avec les isocyanates sur la matière réactive avec les isocyanates, et la somme des ingrédients (i) et (ii) constitue au moins 30% en poids, par rapport au poids total de la composition.

2. Composition selon la revendication 1, qui contient aussi des additifs normaux choisis dans le groupe constitué par les charges, les pigments, la benzoïne, les agents de régulation de l'écoulement et les catalyseurs organo-stanneux.

3. Composition selon la revendication 1 ou 2, dans laquelle la substance réactive avec les isocyanates contient des groupes réactifs choisis parmi les groupes hydroxy, amino et mercapto, et leurs mélanges.

4. Composition selon la revendication 3, dans laquelle la substance réactive avec les isocyanates contient plusieurs groupes hydroxy.

5. Composition selon la revendication 4, dans laquelle la substance réactive avec les isocyanates est choisie parmi une résine acrylique à fonctions hydroxy et une résine polyester à terminaison hydroxy.

6. Composition selon l'une quelconque des revendications 1 à 5, dans laquelle la substance réactive avec les isocyanates possède une masse moléculaire de 100 à 50 000.

7. Composition selon l'une quelconque des revendications 1 à 6, dans laquelle la substance polyfonctionnelle solide en poudre, réactive avec les isocyanates, est solide jusqu'à 50°C et devient fluide dans une gamme de température de 100°C à 150°C.

8. Procédé de revêtement d'un substrat par mise en contact du substrat avec une composition de revêtement en poudre, suivie du thermodurcissement de ladite composition, caractérisé en ce que la composition de revêtement en poudre est une composition de revêtement en poudre selon l'une quelconque des revendications 1 à 7.

9. Composé d'addition de méta- et/ou de para-tétraméthylxylylène diisocyanate avec deux équivalents d'ε-caprolactame.
